# EUROPEAN PATENT APPLICATION

(11) **EP 4 636 457 A1**
(43) Date of publication of application: **22.10.2025**
(21) Application number: 23889835.7
(22) Date of filing: 12.10.2023
(51) Int. Cl.: G02B 25/00, G02B 6/06, G02B 21/00, A61B 90/20, A61B 90/25

(54) **EYEPIECE SYSTEM AND SURGICAL MICROSCOPE**

(30) Priority: 10.04.2023 CN 202310384326
(71) Applicant: Anhui Dendrite Optical Technology Co., Ltd., Hefei Cty, Anhui Province (CN)
(72) Inventor: CHEN, Liang, Shanghai 200040 (CN); ZOU, Xiang, Shanghai 200040 (CN)
(74) Representative: Winter, Brandl - Partnerschaft mbB
(86) International application number: PCT/CN2023/124169
(87) International publication number: WO 2024/212458

(57) **Abstract**

The present invention relates to the technical field of optical microscopes. An eyepiece system and a surgical microscope are provided. The eyepiece system includes a tube lens, a relay lens set disposed in light emergent direction of the tube lens, an eyepiece lens disposed in light emergent direction of the relay lens set, and the relay lens set is connected to the eyepiece lens via a bundle of image-carrying fibers to enable distance and deflection angle between the eyepiece lens and the relay lens set to be continuously adjustable. In the eyepiece system, distance and deflection angle between the eyepiece lens and the relay lens set are continuously adjustable with the arrangement of the bundle of image-carrying fibers connecting the relay lens set and the eyepiece lens; the structure of the eyepiece system can be simplified while the imaging quality is guaranteed, thereby reducing the difficulty of making the eyepiece system and the cost of making the eyepiece system. In case of applying the eyepiece system to manufacture an optical device, the eyepiece lens can be away from the main structure of the optical device by adjusting the length of the bundle of image-carrying fibers, so that the eyepiece lens can be mounted in a position where a user can conveniently observe.

## Description

### TECHNICAL FIELD

The present invention relates to the technical field of optical microscopes, and more particularly, relates to an eyepiece system and a surgical microscope.

### BACKGROUND

The imaging system of surgical microscope usually consists of objective lens, continuous zoom piece and eyepiece system. The eyepiece system is used to receive image generated by the objective lens and magnify it again, so the quality of the eyepiece system has an important impact on the imaging of the whole surgical microscope. The eyepiece system requires adjustable pupil distance, angle variable from 0 to 190 degrees, etc., which poses high challenge to the manufacturing process of the eyepiece system. To achieve the imaging quality requirements, the positional relationship of the object and the image, the straightness of the optical axis, and the positional relationship of the lenses must be controlled within certain tolerances. In related technology, the eyepiece system is set up with multiple prisms and needs to be rotated significantly, which leads to the complexity of the manufacturing process and high cost of manufacture of the eyepiece system.

Therefore, the existing technology needs to be improved and developed.

### SUMMARY

In view of the above deficiencies of the related technology, the present invention provides an eyepiece system and a surgical microscope, which may solve the problems of complicated manufacturing process and high manufacturing cost of existing eyepiece system.

Technical solution of the present invention is as follows.

In a first aspect, the present invention provides an eyepiece system. The eyepiece system includes a tube lens, a relay lens set disposed in a light emergent direction of the tube lens, an eyepiece lens disposed in a light emergent direction of the relay lens set, where the relay lens set is connected to the eyepiece lens via a bundle of image-carrying fibers so that a distance and a deflection angle between the eyepiece lens and the relay lens set are continuously adjustable.

Optionally, the relay lens set includes a first relay lens and a second relay lens, the first relay lens is disposed on a side next to the tube lens, and the bundle of image-carrying fibers has one end fixedly connected to the second relay lens and another end fixedly connected to the eyepiece lens.

Optionally, one end of the bundle of image-carrying fibers is fixed at an image plane of the second relay lens and the other end of the bundle of image-carrying fibers is fixed at a focal plane of the eyepiece lens.

Optionally, the tube lens, the first relay lens and the second relay lens are arranged along an identical optical axis.

In a second aspect, the present invention provides a surgical microscope, the surgical microscope including an imaging system and two eyepiece systems as described in the first aspect. The two eyepiece systems are disposed side-by-side in a light emergent direction of the imaging system. The tube lenses are disposed close to the imaging system. Two parallel beams of light, outputted by the imaging system, correspondingly reach both eyes of a user through the two eyepiece systems respectively.

Optionally, the imaging system includes an objective lens and two continuous zoom pieces disposed side-by-side in a light emergent direction of the objective lens. The tube lenses of the two eyepiece systems are disposed correspondingly in light emergent directions of the two continuous zoom pieces.

Optionally, the bundle of image-carrying fibers is integrated by multiple image-carrying fibers, a diameter of the image-carrying fibers satisfies Ø ≤ M/21, where *Ø* is the diameter of the image-carrying fibers, *l* is an object transfer function of the surgical microscope, and M is a magnification from the objective lens to the tube lens.

Optionally, the continuous zoom piece is set along an identical optical axis as the tube lens and the relay lens set of a correspondingly arranged eyepiece system.

Optionally, the tube lenses and the relay lens sets of the two eyepiece systems and the imaging system are integrable into an optical module.

Optionally, a distance between the bundles of image-carrying fibers of the two eyepiece systems is continuously adjustable to match a pupil distance of the user.

Beneficial effects are as follows. In the eyepiece system of the present invention, the distance and deflection angle between the eyepiece lens and the relay lens set are continuously adjustable with the arrangement of the bundle of image-carrying fibers connecting the relay lens set and the eyepiece lens; the structure of the eyepiece system can be simplified while ensuring the imaging quality, so as to reduce the difficulty of making the eyepiece system and the cost of making the eyepiece system. Moreover, in case of applying the eyepiece system of the present invention to manufacture an optical device (especially a surgical microscope), the eyepiece lens can be away from the main structure of the optical device by adjusting the length of the bundle of image-carrying fibers, so that the eyepiece lens can be mounted in a position where a user can conveniently observe, thus avoiding limitation in the selection of observation positions of the user when using an existing optical device whose eyepiece lens can only be mounted on the top of the main structure of the optical device; in addition, the eyepiece system is easier to integrate.

### BRIEF DESCRIPTION OF THE DRAWINGS

FIG.1 illustrates a schematic structure and an optical path of an eyepiece system provided by an embodiment of the present invention;
FIG.2 is a schematic structural diagram of a cross section of a bundle of image-carrying fibers shown in FIG. 1; and
FIG.3 illustrates a schematic structure and an optical path of a surgical microscope provided by an embodiment of the present invention.

Numerical references: 10 denotes eyepiece system, 11 denotes tube lens, 12 denotes relay lens set, 121 denotes first relay lens, 122 denotes second relay lens, 13 denotes a bundle of image-carrying fibers, 131 denotes image-carrying fiber, 14 denotes eyepiece lens, 20 denotes imaging system, 21 denotes objective lens, 22 denotes continuous zoom piece, and 30 denotes object surface of object.

### DETAILED DESCRIPTION OF THE EMBODIMENTS

An eyepiece system and a surgical microscope are provided according to embodiments of the present invention. The present invention is further detailed hereinafter to make the objects, technical solutions and effects of the present invention clearer and more definite. It should be understood that specific embodiments described herein are used only to explain the present invention rather than to limit the present invention.

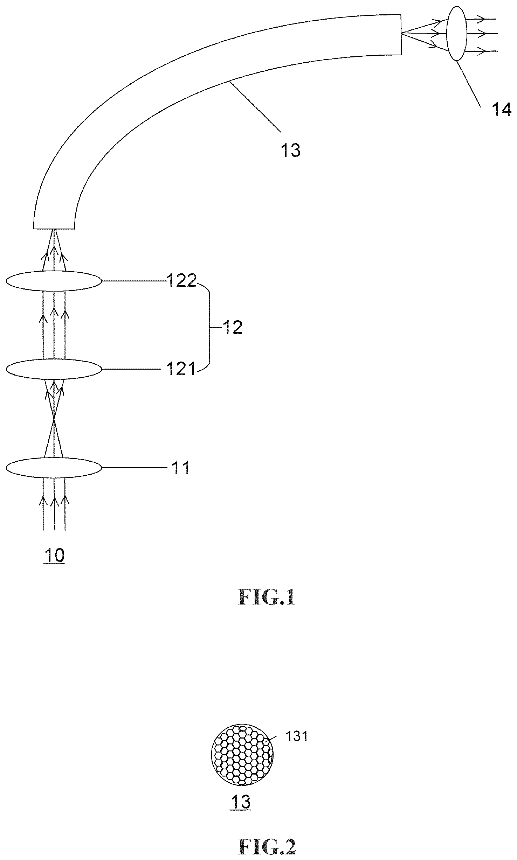

In a first aspect, an eyepiece system is provided according to an embodiment of the present invention. Reference may be made to FIG.1, which illustrates a schematic structure and an optical path of the eyepiece system provided by the embodiment of the present invention. The eyepiece system 10 may include: a tube lens 11, a relay lens set 12 and an eyepiece lens 14. The relay lens set 12 is disposed in a light emergent direction of the tube lens 11, the eyepiece lens 14 is disposed in a light emergent direction of the relay lens set 12, and the relay lens set 12 and the eyepiece lens 14 are connected via a bundle of image-carrying fibers 13 so that a distance and a deflection angle between the eyepiece lens 14 and the relay lens set 12 are continuously adjustable.

Specifically, the eyepiece system 10 of the embodiment operates as follows: after rays emitted from an object to be examined are calibrated to be parallel rays, the parallel rays propagate through the tube lens 11 of the eyepiece system 10 to generate an inverted image, then the inverted image is calibrated by the relay lens set 12 (e.g., a single-magnification imaging module consisting of a first relay lens 121 and a second relay lens 122) to generate an erect image. The erect image is transmitted into the eyepiece lens 14 via the bundle of image-carrying fibers 13, so a user can observe the erect image of the object to be examined through the eyepiece lens 14.

Reference may be made to FIG.2, which is a schematic structural diagram of a cross section of the bundle of image-carrying fibers shown in FIG.1. In the embodiment of the present invention, the bundle of image-carrying fibers 13 is integrated by multiple image-carrying fibers 131. Due to the very soft texture, the image-carrying fibers can be deflected by a large angle, and therefore can be designed into a mechanical structure (i.e., the bundle of image-carrying fibers 13) whose transversal distance and deflection angle can be adjusted freely; the eyepiece lens 14 is fixed on the mechanical structure. In case of applying the eyepiece system 10 to manufacture an optical device (especially a surgical microscope), the mechanical structure is only responsible for adjusting a pupil distance and an angle of deflection, and is irrelevant with an entire imaging optical path of the optical device, which undoubtedly reduces the difficulty of assembling the eyepiece lens 14 and helps to ensure the manufacturing quality of the optical device.

In some embodiments, the relay lens set 12 includes a first relay lens 121 and a second relay lens 122, the first relay lens 121 is disposed on a side next to the tube lens 11, and the bundle of image-carrying fibers 13 has one end fixedly connected to the second relay lens 122 and another end fixedly connected to the eyepiece lens 14.

Preferably, in some embodiments, one end of the bundle of image-carrying fibers 13 is fixed at an image plane of the second relay lens 122, and the other end of the bundle of image-carrying fibers 13 is fixed at a focal plane of the eyepiece lens 14. In this way, loss of energy of the light propagated through the bundle of image-carrying fibers 13 can be reduced, thereby ensuring the imaging quality.

In some embodiments, the tube lens 11, the first relay lens 121 and the second relay lens 122 are arranged along an identical optical axis. In this way, it is easy to assemble components of the eyepiece system 10 together with each other and it is also beneficial to simplify the overall structure of the eyepiece system 10.

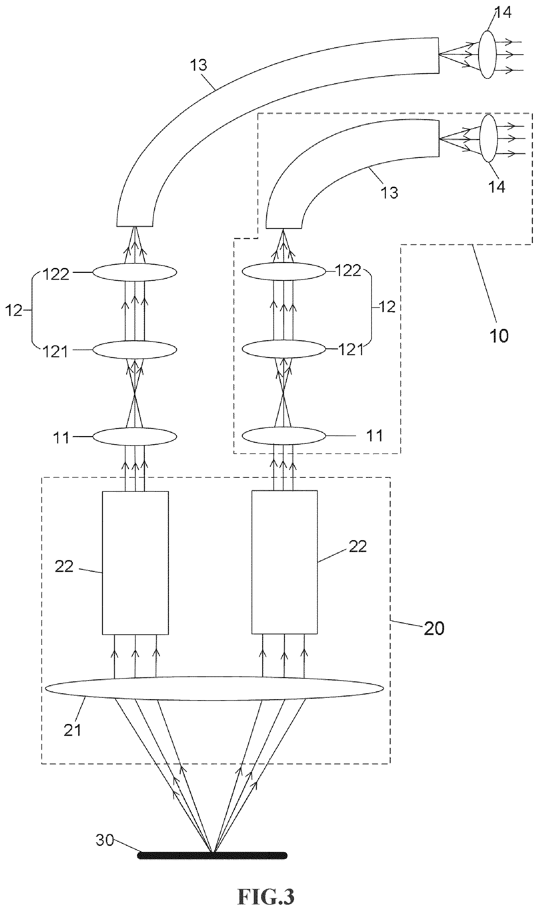

In a second aspect, a surgical microscope is provided according to an embodiment of the present invention. Reference may be made to FIG.3. As shown in FIG.3, the surgical microscope includes an imaging system 20 and two eyepiece systems 10 as described in the embodiment of the first aspect, the two eyepiece systems 10 being disposed side-by-side in a light emergent direction of the the imaging system 20, where the tube lenses 11 are disposed close to the imaging system. Two parallel beams of light, outputted by the imaging system 20, correspondingly reach both eyes of a user through the two eyepiece systems 10 respectively.

In some embodiments, the imaging system 20 includes an objective lens 21, and two continuous zoom pieces 22 disposed side by side in a light emergent direction of the objective lens 21, where the tube lenses 11 of the two eyepiece systems 10 are disposed correspondingly in light emergent directions of the two continuous zoom pieces 22.

Specifically, the surgical microscope of the present embodiment operates as follows: a tissue surface (i.e., object surface) 30 of an object (i.e., the object to be examined) is located in a focal plane of the objective lens 21, light emitted from the object surface 30 is collimated by the objective lens 21 into parallel beams, and the parallel beams outputted from the objective lens 21 are beam-expanded or beam-reduced by the two continuous zoom pieces 22 provided in juxtaposition and then still outputted as parallel beams; the parallel beams output from the continuous zoom pieces 22 respectively enter corresponding eyepiece systems 10, become inverted images after passing through the tube lenses 11 of the eyepiece systems 10, and then are calibrated into erect images by the relay lens sets 12 (e.g., each relay lens set is a single-magnification imaging module composed of a first relay lens 121 and a second relay lens 122); and the erect images are transmitted into the eyepiece lenses 14, so that a user can observe, with his or her eyes via the eyepiece lenses 14, an erect image of the object located under the objective lens 21.

In conjunction with FIG.2, preferably, in some embodiments, the bundle of image-carrying fibers 13 is integrated by multiple image-carrying fibers 131, the diameter of the image-carrying fibers 131 satisfies: Ø ≤ M/2l, where *Ø* is the diameter of the image-carrying fibers, *l* is an object transfer function of the surgical microscope, and M is a magnification from the objective lens 21 to the tube lens 11.

In some embodiments, the continuous zoom piece 22 is disposed along an identical optical axis as the tube lens 11 and the relay lens set 12 of a correspondingly arranged eyepiece system 10. In this way, it is easy to assemble components of the surgical microscope together with each other and it is also beneficial to simplify the overall structure of the surgical microscope.

In some embodiments, the tube lenses 11 and the relay lens sets 12 of the two eyepiece systems 10 and the imaging system 20 may be integrated into an optical module.

In some embodiments, a distance between the bundles of image-carrying fibers 13 of the two eyepiece systems 10 may be continuously adjustable to match a pupil distance of the user.

In summary, in the eyepiece system provided by the present invention, the distance and deflection angle between the eyepiece lens and the relay lens set are continuously adjustable with the arrangement of the bundle of image-carrying fibers connecting the relay lens set and the eyepiece lens; the structure of the eyepiece system can be simplified while the imaging quality is guaranteed, thereby reducing the difficulty of making the eyepiece system and the cost of making the eyepiece system. Moreover, in case of applying the eyepiece system of the present invention to manufacture an optical device (especially a surgical microscope), the eyepiece lens can be away from the main structure of the optical device by adjusting the length of the bundle of image-carrying fibers, so that the eyepiece lens can be mounted in a position where a user can conveniently observe (i.e., convenient for mounting and adjusting), thus avoiding limitation in the selection of observation positions of the user when using an existing optical device whose eyepiece lens can only be mounted on the top of the main structure of the optical device; in addition, the eyepiece system is easier to integrate. Exemplarily, the eyepiece lens is away from the main structure of the surgical microscope and mounted in a position that is convenient for the surgeon to observe or operate, which brings great convenience to surgery.

It should be understood that the application of the present invention is not limited to the foregoing embodiments. The ordinary skilled in the art may make improvements or variations based on the foregoing description, all such improvements and variations shall fall within the scope of protection of appended claims of the present invention.

## Claims

1. An eyepiece system, **characterized by** comprising a tube lens, a relay lens set disposed in a light emergent direction of the tube lens, an eyepiece lens disposed in a light emergent direction of the relay lens set, wherein the relay lens set is connected to the eyepiece lens via a bundle of image-carrying fibers to enable a distance and an angle of deflection between the eyepiece lens and the relay lens set to be continuously adjustable.

2. The eyepiece system of claim 1, wherein the relay lens set comprises a first relay lens and a second relay lens, the first relay lens is disposed on a side next to the tube lens, and the bundle of image-carrying fibers has one end fixedly connected to the second relay lens and another end fixedly connected to the eyepiece lens.

3. The eyepiece system of claim 2, wherein one end of the bundle of image-carrying fibers is fixed at an image plane of the second relay lens and the other end of the bundle of image-carrying fibers is fixed at a focal plane of the eyepiece lens.

4. The eyepiece system of claim 2 or 3, wherein the tube lens, the first relay lens and the second relay lens are arranged along an identical optical axis.

5. A surgical microscope, **characterized by** comprising an imaging system and two eyepiece systems of any one of claims 1-4, the two eyepiece systems being disposed side-by-side in a light emergent direction of the imaging system, wherein the tube lenses are disposed close to the imaging system, and two parallel beams of light, outputted by the imaging system, correspondingly reach both eyes of a user through the two eyepiece systems respectively.

6. The surgical microscope of claim 5, wherein the imaging system comprises an objective lens and two continuous zoom pieces disposed side-by-side in a light emergent direction of the objective lens, and the tube lenses of the two eyepiece systems are disposed correspondingly in light emergent directions of the two continuous zoom pieces.

7. The surgical microscope of claim 6, wherein the bundle of image-carrying fibers is integrated by a plurality of image-carrying fibers, a diameter of the image-carrying fibers satisfies Ø ≤ M/21 , where *Ø* is the diameter of the image-carrying fibers, *l* is an object transfer function of the surgical microscope, and M is a magnification from the objective lens to the tube lens.

8. The surgical microscope of claim 6, wherein the continuous zoom piece is set along an identical optical axis as the tube lens and the relay lens set of a correspondingly arranged eyepiece system.

9. The surgical microscope of claim 5, wherein the tube lenses and the relay lens sets of the two eyepiece systems and the imaging system are integrable into an optical module.

10. The surgical microscope of any one of claims 5-9, wherein a distance between the bundles of image-carrying fibers of the two eyepiece systems is continuously adjustable to match a pupil distance of the user.
